# EUROPÄISCHE PATENTSCHRIFT

(11) **EP 3 613 486 B1**
(45) Veröffentlichungstag und Bekanntmachung des Hinweises auf die Patenterteilung: **07.10.2020**
(21) Anmeldenummer: 18190725.4
(22) Anmeldetag: 24.08.2018
(51) Int. Cl.: B01D 15/38, A61K 38/18, C07K 14/505, B01D 15/36

(54) **VERFAHREN UND ANLAGE ZUR REINIGUNG VON EPO UND/ODER EINEM EPO-DERIVAT**
METHOD AND INSTALLATION FOR THE PURIFICATION OF EPO AND/OR AN EPO DERIVATIVE
PROCÉDÉ ET INSTALLATION DE NETTOYAGE D'EPO ET / OU D'UN DÉRIVÉ D'EPO

(43) Veröffentlichungstag der Anmeldung: 26.02.2020
(73) Patentinhaber: UGA Biopharma GmbH, 16761 Hennigsdorf (DE)
(72) Erfinder: Heßler, Jenny, 12555 Berlin (DE); Hannemann, Wiebke, 15848 Rietz-Neuendorf (DE)
(74) Vertreter: Pfenning, Meinig & Partner mbB

(56) Entgegenhaltungen:
- EP-A1- 0 358 463
- EP-A1- 2 415 779
- EP-A2- 1 064 951
- WO-A1-96/35718
- JP-A- H01 165 393
- US-A1- 2007 293 420
- US-A1- 2012 264 688
- DATABASE WPI Week 200966 Thomson Scientific, London, GB; AN 2009-K48348 XP002788721, & KR 100 900 013 B1 (CJ CHEILJEDANG CORP) 29. Mai 2009 (2009-05-29)

## Beschreibung

Es wird ein Verfahren und Anlage zur Reinigung von EPO und/oder einem EPO-Derivat bereitgestellt. In dem Verfahren wird eine feste Phase zur Immobilisierung von EPO und/oder einem EPO-Derivat eingesetzt, die einen 1-Amino-2-Sulfo-Anthrachinon-Farbstoff gebunden hat. Es wurde gefunden, dass sich durch diese Maßnahme ein starker Reinigungserfolg erzielen lässt, sodass die Aufreinigung von EPO und/oder einem EPO-Derivat zu einer gewünschten Reinheit möglich ist. Es wurde gefunden, dass die sonst gängige Durchführung einer Größenausschluss-Chromatographie, Durchführung einer Umkehrphasen-Chromatographie und/oder Zugabe von Additiven in die Reinigungsflüssigkeiten nicht mehr nötig sind, um die gewünschte Reinheit zu erzielen. Das EPO und/oder ein EPO-Derivat kann damit auf eine schnellere, einfachere, effizientere, kostengünstigere und kontinuierliche Art und Weise zu einem hohen Reinheitsgrad aufgereinigt werden. Mit der erfindungsgemäßen Anlage kann das Verfahren auch automatisch durchgeführt werden.

Zur Aufreinigung von Erythropoetin (EPO) und/oder Derivaten hiervon (EPO-Derivate) wie beispielsweise Darbepoetin α sind im Stand der Technik Reinigungsverfahren bekannt, die mindestens vier Reinigungsschritte aufweisen (z.B. EP 3 075 740 A1). In den bekannten Verfahren wird eine Größenausschluss-Chromatographie (engl. "SEC") und/oder eine Umkehrphasen-Chromatographie (engl. "RP") verwendet bzw. zur Verbesserung des Reinigungsergebnisses Puffer eingesetzt, die L-Arginin enthalten.

SEC und/oder RP haben den Nachteil, dass sie für eine schnelle, effiziente und kostengünstige Reinigung von EPO und/oder EPO-Derivaten im großen Maßstab ungeeignet sind, da sie nur sehr geringe Ladekapazitäten und lange Laufzeiten aufweisen (SEC und RP). Die RP hat den zusätzlichen Nachteil, dass sie regelmäßig organische Lösungsmittel im Laufpuffer erfordert, was im großtechnischen Maßstab kostenintensiv und umweltbelastend ist und zudem ein Sicherheitsrisiko (Explosionsgefahr) darstellt.

Die US 2007/293420 A1 offenbart ein Verfahren zur Reinigung von rekombinantem Erythropoetin, das als chromatographische Reinigungsschritte mindestens eine erste Anionenaustauschchromatographie, eine Affinitätschromatographie, eine Chromatographie mit hydrophober Wechselwirkung, eine Hydroxyapatit-Chromatographie und eine weitere Anionenaustauschchromatographie umfasst.

Die KR 100 900 013 B1 offenbart ein Verfahren zur Reinigung von rekombinantem Erythropoetin, das als chromatographische Reinigungsschritte mindestens eine Affinitätschromatographie, eine Hydroxyapatit-Chromatographie, eine Anionenaustauschchromatographie und eine Gelfiltrationschromatographie umfasst.

Die WO 96/35718 A1 offenbart ein Verfahren zur Reinigung eines Proteins mit Erythropoietin-Aktivität, das als chromatographische Reinigungsschritte mindestens eine Farbstoffchromatographie, eine erste hydrophobe Chromatographie, eine Chromatographie an Hydroxyapatit, eine weitere hydrophobe Chromatographie und eine Anionenaustauschchromatographie umfasst.

Die EP 1 064 951 A2 offenbart ein Verfahren zur Reinigung von mono-PEG-EPO, das als chromatographische Reinigungsschritte mindestens eine Affinitätschromatographie, eine hydrophobe Chromatographie, eine Chromatographie an Hydroxyapatit, eine Umkehrphasen-Chromatographie und eine Anionenaustauschchromatographie umfasst.

Die EP 2 415 779 A1 offenbart ein Verfahren zur Reinigung von einem Erythropoietin-Derivat, das als chromatographische Reinigungsschritte mindestens eine Anionenaustauschchromatographie, eine Affinitätschromatographie, eine Chromatographie an Hydroxyapatit und eine Kationenaustauschchromatographie umfasst.

Die US 2012/264688 A1 offenbart ein Verfahren zur Reinigung von Erythropoietin, das als chromatographische Reinigungsschritte mindestens eine Affinitätschromatographie, eine Anionenaustauschchromatographie, eine Umkehrphasen-Chromatographie, eine Kationenaustauscherchromatographie und eine Größenausschluss-Chromatographie umfasst.

Die EP 0 358 463 A1 offenbart ein Verfahren zur Reinigung von Erythropoietin, das als chromatographische Reinigungsschritte mindestens eine Affinitätschromatographie mit einem Farbstoff, eine weitere Affinitätschromatographie mit einem Lectin und eine Anionenaustauschchromatographie umfasst.

Die JP H01 165393 A offenbart ein Verfahren zur Reinigung von Erythropoietin, das als chromatographische Reinigungsschritte mindestens eine Affinitätschromatographie, eine Chromatographie an Hydroxyapatit und eine Gelfiltrationschromatographie umfasst.

Ausgehend hiervon war es die Aufgabe der vorliegenden Erfindung, ein Verfahren und eine Anlage bereitzustellen, die eine schnellere, einfachere, effizientere und kostengünstigere Aufreinigung von EPO und/oder einem EPO-Derivat (wie z.B. Darbepoetin a) ermöglicht, wobei die Aufreinigung auch in kontinuierlicher Art und Weise möglich sein soll.

Die Aufgabe wird gelöst durch das Verfahren mit den Merkmalen von Anspruch 1 und der Anlage mit den Merkmalen von Anspruch 8. Die abhängigen Ansprüche zeigen vorteilhafte Weiterbildungen der Erfindung auf.

Erfindungsgemäß wird ein Verfahren zur Reinigung von EPO und/oder einem EPO-Derivat bereitgestellt, umfassend
a) Kontaktieren einer Flüssigkeit enthaltend EPO und/oder ein EPO-Derivat mit einer festen Phase, die einen 1-Amino-2-Sulfo-Anthrachinon-Farbstoff gebunden hat, der 1-Amino-4-[4-[amino]-3-sulfoanilino]-9,10-dioxoanthracene-2-sulfonsäure enthält oder daraus besteht;
b) Waschen der festen Phase mit einer Waschflüssigkeit;
c) Elution des EPO und/oder EPO-Derivats von der festen Phase mit einer Elutionsflüssigkeit;
d) Kontaktieren einer Flüssigkeit, die aus der Elutionsflüssigkeit, die EPO und/oder das EPO-Derivat enthält, hervorgeht und einen Phosphatpuffer mit einem pH-Wert im Bereich von pH 5,5 bis 6,5 enthält, mit einem festen Hydroxyapatitmaterial und Sammeln des Durchlaufs, der das EPO und/oder das EPO-Derivat enthält;
e) Kontaktieren des Durchlaufs, der EPO und/oder das EPO-Derivat enthält, mit einem festen Anionenaustauschermaterial, wodurch das EPO(-Derivat) an das feste Anionenaustauschermaterial bindet;
f) Waschen des festen Anionenaustauschermaterials mit einer Waschflüssigkeit;
g) Elution des EPO und/oder EPO-Derivats von dem festen Anionenaustauschermaterial mit einer Elutionsflüssigkeit;
oder bestehend aus diesen Schritten, wobei in dem Verfahren keine Größenausschluss-Chromatographie durchgeführt wird.

Es wurde durch die Anmelderin überraschend gefunden, dass durch den Einsatz einer festen Phase (wie z.B. Agarose), die einen 1-Amino-2-Sulfo- Anthrachinon-Farbstoff (wie z.B. Cibacron blue von Sigma Aldrich) gebunden hat, eine starke Immobilisierung von EPO und/oder EPO-Derivaten (wie z.B. Darbepoetin a) möglich ist, wodurch sich Verunreinigungen in einer Flüssigkeit enthaltend das EPO und/oder das EPO-Derivat entfernen lassen. Es konnte durch diese Maßnahme ein so starker Reinigungserfolg erzielt werden, dass der sonst im Stand der Technik übliche Einsatz einer Größenausschluss-Chromatographie, einer Umkehrphasen-Chromatographie und/oder teurer Pufferzusätze (z.B. L-Arginin im Puffer) unnötig wird. Der entscheidende Vorteil des Verfahrens liegt somit darin, dass EPO und/oder einem EPO-Derivat auf eine schnellere, einfachere, effizientere und kostengünstigere Art und Weise zu einem gewünschten Grad aufgereinigt werden kann und das Reinigungsverfahren kontinuierlich betrieben werden kann, was einen zusätzlichen Zeit- und Effizienzvorteil bringt.

In der erfindungsgemäßen Ausführungsform des Verfahrens enthält der 1-Amino-2-Sulfo-Anthrachinon-Farbstoff 1-Amino-4-[4-[amino]-3-sulfoanilino]-9,10-dioxoanthracene-2-sulfonsäure oder besteht daraus. Besonders bevorzugt enthält der 1-Amino-2-Sulfo-Anthrachinon-Farbstoff 1-Amino-4-[4-[[4-chloro-6-(2-sulfoanilino)-1,3,5-triazin-2-yl]amino]-3-sulfoanilino]-9,10-dioxoanthracene-2-sulfonsäure (auch bekannt als Cibacron blue, Cibacron blue 3G-A oder Cibacron blue F3G-A) oder besteht daraus.

Der 1-Amino-2-Sulfo-Anthrachinon-Farbstoff kann kovalent an die feste Phase gebunden sein.

Ferner kann der 1-Amino-2-Sulfo-Anthrachinon-Farbstoff über ein Abstandshaltermolekül an die feste Phase gebunden sein. Optional ist das Abstandshaltermolekül über eine Bindung ausgewählt aus der Gruppe bestehend aus Etherbindung, Amidbindung, Thioetherbindung, Hydrazonbindung und Kombinationen hiervon mit der festen Phase verbunden und/oder mit dem 1-Amino-2-Sulfo-Anthrachinon-Farbstoff verbunden. Insbesondere weist das Abstandshaltermolekül mindestens zwei Kohlenstoffatome, bevorzugt zwei bis vier Kohlenstoffatome, und/oder mindestens eine Hydroxygruppe auf (siehe z.B. CaptoBlue® des Herstellers GE Healthcare Life Sciences).

Die feste Phase kann ein Material enthalten oder daraus bestehen, das ausgewählt ist aus der Gruppe bestehend aus Sephadex, Sepharose, Agarose, Polyacrylamid, Methacrylpolymer, Kieselerde und Kombinationen hiervon.

In der erfindungsgemäßen Ausführungsform des Verfahrens wird eine Flüssigkeit enthaltend EPO und/oder das EPO-Derivat nach Schritt c) mit einem festen Hydroxyapatitmaterial kontaktiert und der Durchlauf (d.h. die Flüssigkeit nach der Kontaktierung mit dem Hydroxyapatitmaterial) gesammelt, der das EPO und/oder das EPO-Derivat enthält.

In der erfindungsgemäßen Ausführungsform des Verfahrens umfasst das Verfahren die folgenden Schritte:
i) Kontaktieren einer Flüssigkeit enthaltend EPO und/oder das EPO-Derivat mit einem festen Anionenaustauschermaterial, bevorzugt einem starken Anionenaustauschermaterial, wodurch das EPO-Derivat an das feste Anionenaustauschermaterial bindet;
ii) Waschen des festen Anionenaustauschermaterials mit einer Waschflüssigkeit; und
iii) Elution des EPO und/oder EPO-Derivats von dem festen Anionenaustauschermaterial mit einer Elutionsflüssigkeit.

Erfindungsgemäß erfolgt die Kontaktierung aus Schritt i) zeitlich nach der Kontaktierung der Flüssigkeit enthaltend EPO und/oder ein EPO-Derivat mit dem festen Hydroxyapatitmaterial. In diesem Fall enthält die in Schritt i) eingesetzte Flüssigkeit den Durchlauf nach Kontaktierung mit dem Hydroxyapatitmaterial (d.h. Material, das nach der Kontaktierung mit dem Hydroxyapatitmaterial an dieses nicht gebunden hat).

Die bevorzugteste Ausführungsform des Verfahrens umfasst daher die Schritte
a) Kontaktieren einer Flüssigkeit enthaltend EPO und/oder ein EPO-Derivat mit einer festen Phase, die einen 1-Amino-2-Sulfo-Anthrachinon-Farbstoff gebunden hat, der 1-Amino-4-[4-[amino]-3-sulfoanilino]-9,10-dioxoanthracene-2-sulfonsäure enthält oder daraus besteht;
b) Waschen der festen Phase mit einer Waschflüssigkeit;
c) Elution des EPO und/oder EPO-Derivats von der festen Phase mit einer Elutionsflüssigkeit;
d) Reduktion des Salzgehalts der Elutionsflüssigkeit, bevorzugt über einen Schritt ausgewählt aus der Gruppe bestehend aus Dialyse (z.B. Elektrodialyse), Verdünnung, Nanofiltration und Kombinationen hiervon;
e) Kontaktieren der salzreduzierten Elutionsflüssigkeit mit einem festen Hydroxyapatitmaterial und Sammeln des Durchlaufs;
f) Kontaktieren des (optional pH-korrigierten) Durchlaufs mit einem festen Anionenaustauschermaterial, bevorzugt einem starken Anionenaustauschermaterial, wodurch das EPO-Derivat an das feste Anionenaustauschermaterial bindet;
g) Waschen des festen Anionenaustauschermaterials mit einer Waschflüssigkeit; und
h) Elution des EPO und/oder EPO-Derivats von dem festen Anionenaustauschermaterial mit einer weiteren Elutionsflüssigkeit,
wobei in dem Verfahren keine Größenausschluss-Chromatographie durchgeführt wird.

Optional wird vor dem Kontaktieren der eluierten Elutionsflüssigkeit mit dem festen Anionenaustauschermaterial der pH-Wert des Durchlaufs angepasst (bzw. korrigiert), bevorzugt auf einen neutralen pH-Wert, bevorzugt auf einen pH-Wert von 6,5 bis 7,5, insbesondere auf einen pH-Wert von 7,0. Die Anpassung des pH-Werts kann durch Zugabe von Säure und/oder einer Lauge zu dem Durchlauf aus Schritt e) erreicht werden. Die Anpassung des pH-Werts kann im Rahmen einer Umpufferung stattfinden, wobei die Umpufferung bevorzugt über einen Schritt ausgewählt aus der Gruppe bestehend aus Dialyse (z.B. Elektrodialyse), Verdünnung, Nanofiltration und Kombinationen hiervon, durchgeführt wird. Optional kann die bevorzugteste Ausführungsform des Verfahrens auch aus den genannten Schritten bestehen, d.h. keine weiteren Verfahrensschritte aufweisen. Insbesondere ist der 1-Amino-2-Sulfo-Anthrachinon-Farbstoff dazu geeignet, über nicht-kovalente Wechselwirkungen an EPO und/oder das EPO-Derivat zu binden.

Das Verfahren kann ferner dadurch gekennzeichnet sein, dass die Flüssigkeit enthaltend EPO und/oder das EPO-Derivat, die Waschflüssigkeit und/oder die Elutionsflüssigkeit eine Puffersubstanz enthält, bevorzugt einen Phosphatpuffer. Ferner kann die die Flüssigkeit enthaltend EPO und/oder das EPO-Derivat, die Waschflüssigkeit und/oder die Elutionsflüssigkeit einen pH-Wert im Bereich von 5,0 bis 9,0, bevorzugt im Bereich von 5,5 bis 8,5, besonders bevorzugt im Bereich von 6,0 bis 8,0, insbesondere im Bereich von 6,5 bis 7,5, aufweisen. In einer bevorzugten Ausführungsform des erfindungsgemäßen Verfahrens enthält die Flüssigkeit enthaltend EPO und/oder das EPO-Derivat, die Waschflüssigkeit und/oder die Elutionsflüssigkeit maximal 10 mM L-Arginin (bevorzugt kein L-Arginin), insbesondere jede Flüssigkeit der Anlage maximal 10 mM L-Arginin (bevorzugt kein L-Arginin).

Beim Kontaktieren der Flüssigkeit enthaltend EPO und/oder ein EPO-Derivat mit der festen Phase enthaltend den 1-Amino-2-Sulfo-Anthrachinon-Farbstoff kann die Flüssigkeit und/oder die Waschflüssigkeit einen Phosphatpuffer enthalten. Die Phosphationenkonzentration kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit und/oder die Waschflüssigkeit kein nicht-pufferndes Salz (wie z.B. NaCl und/oder KCl). Der pH-Wert des Puffers liegt bevorzugt im Bereich von pH 5,0 bis 9,0, bevorzugt im Bereich von pH 5,5 bis 8,5, besonders bevorzugt im Bereich von pH 6,0 bis 8,0, ganz besonders bevorzugt im Bereich von pH 6,5 bis 7,5, insbesondere bei pH 7,0. Entsprechendes kann für die Elutionsflüssigkeit gelten, wobei die Elutionsflüssigkeit bevorzugt ein nicht-pufferndes Salz (z.B. NaCl und/oder KCl) enthält, besonders bevorzugt in einer Konzentration von 0,5 M bis 2 M, ganz besonders bevorzugt 1,0 M bis 2,0 M, insbesondere 1,5 M bis 2,0 M.

Beim Kontaktieren der Flüssigkeit enthaltend EPO und/oder ein EPO-Derivat (erfindungsgemäß geht diese Flüssigkeit aus dem Eluat von der festen Phase enthaltend den 1-Amino-2-Sulfo-Anthrachinon-Farbstoff hervor) mit einem festen Hydroxyapatitmaterial enthält die Flüssigkeit einen Phosphatpuffer. Die Phosphationenkonzentration kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit kein nicht-pufferndes Salz (wie z.B. NaCl und/oder KCl). Der pH-Wert des Puffers liegt im Bereich von pH 5,5 bis 6,5, insbesondere bei pH 6,0. Die besten Reinigungsergebnisse werden bei pH 6,0 erzielt.

Beim Kontaktieren der Flüssigkeit enthaltend EPO und/oder ein EPO-Derivat (erfindungsgemäß geht diese Flüssigkeit aus dem Durchlauf nach der Kontaktierung mit dem Hydroxyapatitmaterial hervor) mit dem festen Anionenaustauschermaterial kann die Flüssigkeit und/oder die Waschflüssigkeit einen Trispuffer (oder Phosphatpuffer) enthalten. Die Trislonenkonzentration (oder Phosphatkonzentration) kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit und/oder die Waschflüssigkeit kein nicht-pufferndes Salz (wie z.B. NaCl und/oder KCl). Der pH-Wert des Puffers liegt bevorzugt im Bereich von pH 5,0 bis 9,0, bevorzugt im Bereich von pH 5,5 bis 8,5, besonders bevorzugt im Bereich von pH 6,0 bis 8,0, ganz besonders bevorzugt im Bereich von pH 6,5 bis 7,5, insbesondere bei pH 7,0. Entsprechendes kann für die Elutionsflüssigkeit gelten, wobei die Elutionsflüssigkeit bevorzugt ein nicht-pufferndes Salz (z.B. NaCl und/oder KCl) enthält, besonders bevorzugt in einer Konzentration von 0,1 M bis 2 M, ganz besonders bevorzugt 0,2 M bis 1,0 M, insbesondere 0,4 M bis 0,6 M.

Ferner kann die Flüssigkeit enthaltend EPO und/oder das EPO-Derivat eine Fermentationsbrühe enthalten oder daraus bestehen, bevorzugt einen Überstand einer Fermentation mit biologischen Zellen, die das EPO und/oder EPO-Derivat produzieren. Insbesondere wird die Fermentationsbrühe über eine fluidische Verbindung zwischen dem Fermenter und der festen Phase vom Fermenter zur festen Phase geleitet.

Erfindungsgemäß wird in dem Verfahren keine Größenausschluss-Chromatographie durchgeführt. In einer bevorzugten Ausführungsform des Verfahrens wird keine Umkehrphasen-Chromatographie durchgeführt.

Es wird ferner eine Anlage zur Reinigung von EPO und/oder einem EPO-Derivat bereitgestellt, enthaltend
a) eine feste Phase, optional in einer Säule enthalten, wobei die feste Phase einen 1-Amino-2-Sulfo- Anthrachinon-Farbstoff gebunden hat, der 1-Amino-4-[4-[amino]-3-sulfoanilino]-9,10-dioxoanthracene-2-sulfonsäure enthält oder daraus besteht;
b) eine Flüssigkeit enthaltend EPO und/oder ein EPO-Derivat, die fluidisch, bevorzugt über ein Ventil, mit der festen Phase verbunden ist;
c) eine Waschflüssigkeit, die fluidisch, bevorzugt über ein Ventil, mit der festen Phase verbunden ist;
d) eine Elutionsflüssigkeit, die fluidisch, bevorzugt über ein Ventil, mit der festen Phase verbunden ist;
e) ein festes Hydroxyapatitmaterial;
f) ein festes Anionenaustauschermaterial;
g) eine Pumpe, die dazu geeignet ist, eine Flüssigkeit ausgewählt aus der Gruppe bestehend aus Flüssigkeit enthaltend das EPO-Derivat, Waschflüssigkeit, Elutionsflüssigkeit und Mischungen und Kombinationen hiervon, mit der festen Phase zu kontaktieren; und
h) eine Steuereinheit, die dazu konfiguriert ist, zunächst die Flüssigkeit enthaltend das EPO-Derivat mit der festen Phase zu kontaktieren, anschließend die Waschflüssigkeit mit der festen Phase zu kontaktieren und dann die Elutionsflüssigkeit mit der festen Phase zu kontaktieren und das Eluat zu sammeln, wobei die Steuereinheit ferner dazu konfiguriert ist, eine Flüssigkeit, die fluidisch mit dem festen Hydroxyapatitmaterial verbunden ist, aus dem Eluat hervorgeht und einen Phosphatpuffer mit einem pH-Wert im Bereich von pH 5,5 bis 6,5 enthält, mit dem festen Hydroxyapatitmaterial zu kontaktieren und den Durchlauf zu sammeln, der EPO und/oder das EPO-enthält, sowie den Durchlauf, der fluidisch mit dem festen Anionenaustauschermaterial verbunden ist, mit dem festen Anionenaustauschermaterial zu kontaktieren, das feste Anionenaustauschermaterial mit einer Waschflüssigkeit, die fluidisch mit dem festen Anionenaustauschermaterial verbunden ist, zu waschen und mit einer Elutionsflüssigkeit, die fluidisch mit dem festen Anionenaustauschermaterial verbunden ist, das EPO und/oder EPO-Derivat von dem festen Anionenaustauschermaterial zu eluieren;
oder bestehend daraus, wobei die Anlage keine feste Phase zur Größenausschluss-Chromatographie enthält.

Der mit festen Phase der Anlage erzielbare Reinigungserfolg ist so hoch, dass es unnötig wird, die Anlage mit einer Größenausschluss-Chromatographie, einer Umkehrphasen-Chromatographie und/oder Flüssigkeiten mit teuren Inhaltsstoffen (wie z.B. L-Arginin) auszustatten. Dadurch erlaubt es die Anlage, EPO und/oder ein EPO-Derivat (wie z.B. Darbepoetin a) auf schnellere, einfachere, effizientere und kostengünstigere Art und Weise zu einem gewünschten Grad aufzureinigen. Ferner erlaubt es die Anlage, die Aufreinigung (von einer Fermentationsbrühe mit EPO und/oder EPO-Derivat bis zum gereinigten EPO und/oder EPO-Derivat) kontinuierlich durchzuführen, was einen zusätzlichen Zeit- und Effizienzvorteil bringt.

Die Anlage ist erfindungsgemäß dadurch gekennzeichnet, dass der 1-Amino-2-Sulfo-Anthrachinon-Farbstoff 1-Amino-4-[4-[amino]-3-sulfoanilino]-9,10-dioxoanthracene-2-sulfonsäure enthält oder daraus besteht, besonders bevorzugt 1-Amino-4-[4-[[4-chloro-6-(2-sulfoanilino)-1,3,5-triazin-2-yl]amino]-3-sulfoanilino]-9,10-dioxoanthracene-2-sulfonsäure (auch bekannt als Cibacron blue, Cibacron blue 3G-A oder Cibacron blue F3G-A) enthält oder daraus besteht.

Der 1-Amino-2-Sulfo-Anthrachinon-Farbstoff kann kovalent an die feste Phase (z.B. Agarose) gebunden sein.

Ferner kann der 1-Amino-2-Sulfo-Anthrachinon-Farbstoff über ein Abstandshaltermolekül an die feste Phase gebunden sein. Optional ist das Abstandshaltermolekül über eine Bindung ausgewählt aus der Gruppe bestehend aus Etherbindung, Amidbindung, Thioetherbindung, Hydrazonbindung und Kombinationen hiervon mit der festen Phase verbunden und/oder mit dem 1-Amino-2-Sulfo-Anthrachinon-Farbstoff verbunden. Insbesondere weist das Abstandshaltermolekül mindestens zwei Kohlenstoffatome, bevorzugt zwei bis vier Kohlenstoffatome, und/oder mindestens eine Hydroxygruppe auf (siehe z.B. CaptoBlue® des Herstellers GE Healthcare Life Sciences).

Die feste Phase kann ein Material enthalten oder daraus bestehen, das ausgewählt ist aus der Gruppe bestehend aus Sephadex, Sepharose, Agarose, Polyacrylamid, Methacrylpolymer, Kieselerde und Kombinationen hiervon.

In der erfindungsgemäßen Ausgestaltungsform enthält die Anlage ein festes Hydroxyapatitmaterial (optional in einer Säule enthalten) und die Steuereinheit ist dazu konfiguriert ist, eine Flüssigkeit enthaltend EPO und/oder das EPO-Derivat nach Schritt c), mit dem festen Hydroxyapatitmaterial zu kontaktieren und den EPO und/oder das EPO-Derivat enthaltenden Durchlauf zu sammeln. Hierzu ist die Flüssigkeit enthaltend EPO und/oder das EPO-Derivat fluidisch mit dem festen Hydroxyapatitmaterial verbunden.

In der erfindungsgemäßen Ausgestaltungsform enthält die Anlage ein festes Anionenaustauschermaterial (optional in einer Säule enthalten), bevorzugt ein starkes Anionenaustauschermaterial, und die Steuereinheit der Anlage ist dazu konfiguriert ist, die folgenden Schritte durchzuführen:
i) Kontaktieren einer Flüssigkeit enthaltend EPO und/oder ein EPO-Derivat mit dem festen Anionenaustauschermaterial, wobei die Flüssigkeit enthaltend EPO und/oder ein EPO-Derivat fluidisch, bevorzugt über ein Ventil, mit dem festen Anionenaustauschermaterial verbunden ist;
ii) Waschen des festen Anionenaustauschermaterials mit einer Waschflüssigkeit, wobei die Waschflüssigkeit fluidisch, bevorzugt über ein Ventil, mit dem festen Anionenaustauschermaterial verbunden ist;
iii) Elution des EPO und/oder EPO-Derivats von dem festen Anionenaustauschermaterial mit einer Elutionsflüssigkeit, wobei die Elutionsflüssigkeit fluidisch, bevorzugt über ein Ventil, mit dem festen Anionenaustauschermaterial verbunden ist.

In der erfindungsgemäßen Ausführungsform ist die Steuereinheit dazu konfiguriert, die Kontaktierung aus Schritt i) zeitlich nach der Kontaktierung der Flüssigkeit enthaltend EPO und/oder das EPO-Derivat mit dem festen Hydroxyapatitmaterial durchzuführen. In diesem Fall enthält die in Schritt i) eingesetzte Flüssigkeit den Durchlauf nach Kontaktierung mit dem Hydroxyapatitmaterial (d.h. Material, das nach der Kontaktierung mit dem Hydroxyapatitmaterial an dieses nicht gebunden hat).

In der bevorzugtesten Ausführungsform enthält die Anlage somit
a) eine feste Phase, die einen 1-Amino-2-Sulfo- Anthrachinon-Farbstoff gebunden hat, optional in einer Säule enthalten, wobei die feste Phase einen 1-Amino-2-Sulfo- Anthrachinon-Farbstoff gebunden hat, der 1-Amino-4-[4-[[4-chloro-6-(2-sulfoanilino)-1,3,5-triazin-2-yl]amino]-3-sulfoanilino]-9,10-dioxoanthracene-2-sulfonsäure enthält oder daraus besteht;
b) ein festes Hydroxyapatitmaterial, optional in einer Säule enthalten;
c) ein festes Anionenaustauschermaterial, bevorzugt ein starkes Anionenaustauschermaterial, optional in einer Säule enthalten;
d) eine Flüssigkeit enthaltend EPO und/oder ein EPO-Derivat, die fluidisch, bevorzugt über ein Ventil, mit der festen Phase enthaltend den 1-Amino-2-Sulfo- Anthrachinon-Farbstoff verbunden ist;
e) eine Waschflüssigkeit, die fluidisch, bevorzugt über ein Ventil, mit der festen Phase enthaltend den 1-Amino-2-Sulfo- Anthrachinon-Farbstoff verbunden ist;
f) eine Elutionsflüssigkeit, die fluidisch, bevorzugt über ein Ventil, mit der festen Phase enthaltend den 1-Amino-2-Sulfo- Anthrachinon-Farbstoff verbunden ist;
g) eine zweite Waschflüssigkeit, die fluidisch, bevorzugt über ein Ventil, mit dem festen Anionenaustauschermaterial verbunden ist;
h) eine zweite Elutionsflüssigkeit, die fluidisch, bevorzugt über ein Ventil, mit dem festen Anionenaustauschermaterial verbunden ist;
i) eine fluidische Verbindung zwischen der festen Phase enthaltend den 1-Amino-2-Sulfo-Anthrachinon-Farbstoff und dem festen Hydroxyapatitmaterial, bevorzugt über eine Einheit zur Reduktion des Salzgehalts, bevorzugt eine Einheit ausgewählt aus der Gruppe bestehend aus Dialyseeinheit (z.B. Elektrodialyseeinheit), Verdünnungseinheit, Nanofiltrationseinheit und Kombinationen hiervon;
j) eine fluidische Verbindung zwischen dem festen Hydroxyapatitmaterial und dem festen Anionenaustauschermaterial, bevorzugt über eine Einheit zur Anpassung des pH-Werts, die bevorzugt konfiguriert ist zur Anpassung des pH-Werts auf einen neutralen pH-Wert, besonders bevorzugt auf einen pH-Wert von 6,5 bis 7,5, insbesondere auf einen pH-Wert von 7,0, wobei die Einheit zur Anpassung des pH-Werts bevorzugt ausgewählt ist aus der Gruppe bestehend aus Einheit zur Zugabe einer Säure und/oder Lauge, Dialyseeinheit (z.B. Elektrodialyseeinheit), Verdünnungseinheit, Nanofiltrationseinheit und Kombinationen hiervon;
k) eine Pumpe, die dazu geeignet ist, eine Flüssigkeit ausgewählt aus der Gruppe bestehend aus Flüssigkeit enthaltend das EPO-Derivat, Waschflüssigkeit, Elutionsflüssigkeit, zweite Waschflüssigkeit, zweite Elutionsflüssigkeit und Mischungen und Kombinationen hiervon, mit der festen Phase enthaltend den 1-Amino-2-Sulfo- Anthrachinon-Farbstoff, dem festen Hydroxyapatitmaterial und dem festen Anionenaustauschermaterial zu kontaktieren;
l) eine Steuereinheit, die dazu konfiguriert ist, zunächst die Flüssigkeit enthaltend das EPO-Derivat mit der festen Phase enthaltend den 1-Amino-2-Sulfo- Anthrachinon-Farbstoff zu kontaktieren, anschließend die Waschflüssigkeit mit der festen Phase zu kontaktieren, dann die Elutionsflüssigkeit mit der festen Phase zu kontaktieren, anschließend die Elutionsflüssigkeit, bevorzugt über eine Umpufferungseinrichtung, mit dem festen Hydroxyapatitmaterial zu kontaktieren, daraufhin die mit dem festen Hydroxyapatitmaterial kontaktierte Flüssigkeit, bevorzugt über eine Umpufferungseinrichtung, mit dem festen Anionenaustauschermaterial zu kontaktieren, anschließend die zweite Waschflüssigkeit mit dem festen Anionenaustauschermaterial zu kontaktieren und dann die zweite Elutionsflüssigkeit mit dem festen Anionenaustauschermaterial zu kontaktieren;
wobei die Anlage keine feste Phase zur Größenausschluss-Chromatographie enthält.

Optional kann die Anlage der bevorzugtesten Ausgestaltungsform auch aus den genannten Komponenten bestehen, d.h. keine weiteren Komponenten aufweisen. Insbesondere ist der 1-Amino-2-Sulfo-Anthrachinon-Farbstoff dazu geeignet, über nicht-kovalente Wechselwirkungen an EPO und/oder das EPO-Derivat zu binden.

Die Einheit zur Reduktion des Salzgehalts kann einen oder mehrere Puffer enthalten, beispielsweise einen 20 mM Phosphatpuffer mit einem pH-Wert von 6,0. Die Einheit zur Anpassung des pH-Werts kann einen oder mehrere Puffer enthalten, beispielsweise einen 20 mM Trispuffer oder Phosphatpuffer mit einem pH-Wert von 7,0.

Die Flüssigkeit enthaltend EPO und/oder das EPO-Derivat, die Waschflüssigkeit und/oder die Elutionsflüssigkeit kann eine Puffersubstanz enthalten, bevorzugt einen Phosphatpuffer. Ferner kann die Flüssigkeit enthaltend EPO und/oder das EPO-Derivat, die Waschflüssigkeit und/oder die Elutionsflüssigkeit einen pH-Wert im Bereich von 5,0 bis 9,0, bevorzugt im Bereich von 5,5 bis 8,5, besonders bevorzugt im Bereich von 6,0 bis 8,0, insbesondere im Bereich von 6,5 bis 7,5, aufweisen. In einer bevorzugten Ausgestaltungsform enthält die Flüssigkeit enthaltend EPO und/oder das EPO-Derivat, die Waschflüssigkeit und/oder die Elutionsflüssigkeit maximal 10 mM L-Arginin (bevorzugt kein L-Arginin), insbesondere jede Flüssigkeit der Anlage maximal 10 mM L-Arginin (bevorzugt kein L-Arginin).

Die Flüssigkeit enthaltend EPO und/oder ein EPO-Derivat und/oder die Waschflüssigkeit, die fluidisch mit der festen Phase enthaltend den 1-Amino-2-Sulfo-Anthrachinon-Farbstoff verbunden sind, kann einen Phosphatpuffer enthalten. Die Phosphationenkonzentration kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit und/oder die Waschflüssigkeit kein nicht-pufferndes Salz (wie z.B. NaCl und/oder KCl). Der pH-Wert des Puffers liegt bevorzugt im Bereich von pH 5,0 bis 9,0, bevorzugt im Bereich von pH 5,5 bis 8,5, besonders bevorzugt im Bereich von pH 6,0 bis 8,0, ganz besonders bevorzugt im Bereich von pH 6,5 bis 7,5, insbesondere bei pH 7,0. Entsprechendes kann für die Elutionsflüssigkeit gelten, die fluidisch mit der festen Phase enthaltend einen 1-Amino-2-Sulfo-Anthrachinon-Farbstoff verbunden ist, wobei die Elutionsflüssigkeit bevorzugt ein nicht-pufferndes Salz (z.B. NaCl und/oder KCl) enthält, besonders bevorzugt in einer Konzentration von 0,5 M bis 2 M, ganz besonders bevorzugt 1,0 M bis 2,0 M, insbesondere 1,5 M bis 2,0 M.

Die Flüssigkeit enthaltend EPO und/oder ein EPO-Derivat, die fluidisch mit dem festen Hydropxyapatitmaterial verbunden ist (erfindungsgemäß geht diese Flüssigkeit aus dem Eluat von der festen Phase enthaltend den 1-Amino-2-Sulfo-Anthrachinon-Farbstoff hervor) enthält einen Phosphatpuffer.

Die Phosphationenkonzentration kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit kein nicht-pufferndes Salz (wie z.B. NaCl und/oder KCl). Der pH-Wert des Puffers liegt im Bereich von pH 5,5 bis 6,5, insbesondere bei pH 6,0. Die besten Reinigungsergebnisse werden bei pH 6,0 erzielt.

Die Flüssigkeit enthaltend EPO und/oder ein EPO-Derivat und/oder die Waschflüssigkeit, die fluidisch mit dem festen Anionenaustauschermaterial verbunden sind (erfindungsgemäß geht diese Flüssigkeit aus dem Durchlauf nach der Kontakierung mit dem Hydroxyapatitmaterial hervor) kann einen Trispuffer (oder Phosphatpuffer) enthalten. Die Tris-Ionenkonzentration (oder Phosphatkonzentration) kann mindestens 5 mM, optional von 5 bis 50 mM, bevorzugt 10 bis 40 mM, besonders bevorzugt 15 bis 25 mM, insbesondere 20 mM, betragen. Insbesondere enthält die Flüssigkeit und/oder die Waschflüssigkeit kein nicht-pufferndes Salz (wie z.B. NaCl und/oder KCl). Der pH-Wert des Puffers liegt bevorzugt im Bereich von pH 5,0 bis 9,0, bevorzugt im Bereich von pH 5,5 bis 8,5, besonders bevorzugt im Bereich von pH 6,0 bis 8,0, ganz besonders bevorzugt im Bereich von pH 6,5 bis 7,5, insbesondere bei pH 7,0. Entsprechendes kann für die Elutionsflüssigkeit gelten, die fluidisch mit dem festen Anionenaustauschermaterial verbunden ist, wobei die Elutionsflüssigkeit bevorzugt ein nicht-pufferndes Salz (z.B. NaCl und/oder KCl) enthält, besonders bevorzugt in einer Konzentration von 0,1 M bis 2 M, ganz besonders bevorzugt 0,2 M bis 1,0 M, insbesondere 0,4 M bis 0,6 M.

Ferner kann die Flüssigkeit enthaltend EPO und/oder das EPO-Derivat eine Fermentationsbrühe enthalten oder daraus bestehen, bevorzugt einen Überstand einer Fermentation mit biologischen Zellen, die das EPO und/oder EPO-Derivat produzieren. Insbesondere weist die Anlage eine fluidische Verbindung zwischen dem Fermenter und der festen Phase auf, die dazu ausgebildet ist, die Fermentationsbrühe vom Fermenter zur festen Phase zu leiten.

In einer bevorzugten Ausgestaltungsform ist die Anlage nicht zur Durchführung einer Umkehrphasen-Chromatographie konfiguriert. Erfindungsgemäß enthält die Anlage keine feste Phase zur Größenausschluss-Chromatographie. Insbesondere enthält die Anlage keine feste Phase zur Umkehrphasen-Chromatographie.

Anhand der nachfolgenden Figur und des nachfolgenden Beispiels soll der erfindungsgemäße Gegenstand näher erläutert werden, ohne diesen auf die hier dargestellten, spezifischen Ausführungsformen einschränken zu wollen.

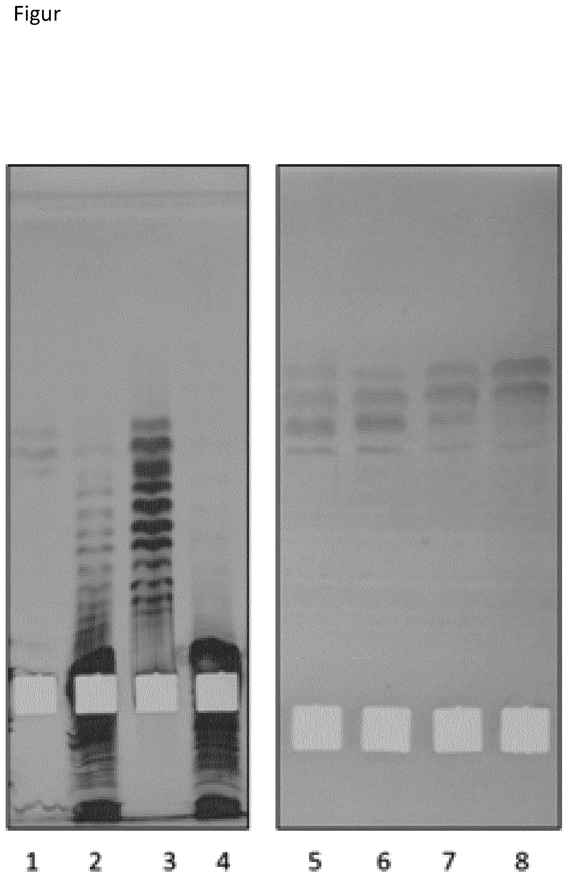

Die Figur zeigt das Ergebnis einer Isoelektrischen Fokusierung (Gelelektrophorese), das beladen wurde mit Darbepoetin α des Herstellers Amgen (Positionen 1 und 5), einem Eluat von der festen Phase mit dem (kovalent) gebundenen 1-Amino-2-Sulfo- Anthrachinon-Farbstoff Cibracron blue (Position 2), einem Durchfluss durch das feste Hydroxyapatitmaterial (Position 3), einem Eluat von dem festen Hydroxyapatitmaterial (Position 4) und eine erste Fraktion (Position 6), einer zweiten Fraktion (Position 7) und einer dritten Fraktion (Position 8) eines Eluats von dem starken Anionenaustauschermaterial. Der Durchfluss durch das feste Hydroxyapatitmaterial (Position 3) besteht fast ausschließlich aus Darbepoetin a. Die hochsialylierten Formen des Darbepoetin α. sind dabei im oberen Teil der Figur und die niedrig sialylierten Formen des Darbepoetin α im unteren Teil der Figur. Die hochsialylierten Formen sind die gewünschten Formen, da ihre Halbwertszeit im Blut höher ist. Im Reinigungsschritt mit dem starken Anionenaustauschermaterial können die niedrigsialylierten Formen von den hochsialylierten Formen abgetrennt werden. Beispielsweise wird 90% des Gesamtproteins aus dem Durchlauf des Hydroxyapatitmaterials über die Trennung mit dem starken Anionenaustauschermaterial entfernt, sodass nur hochsialyliertes Darbepoetin α. Isoliert wird. Das Darbepoetin α des Herstellers Amgen (siehe Position 5) ist hochsialyliertes Darbepoetin α. Folglich weist die an Position 6 im Gel dargestellte Fraktion des über das erfindungsgemäße Verfahren gereinigten Darbepoetin α ebenfalls einen hohen Anteil an hoch-sialyliertem Darbepoeting α auf und kommt dem Sialylierungsprofil von Darbepoeting α des Herstellers Amgen (Positionen 1 und 5) sehr nahe.

### Beispiel - Reinigungsverfahren von Darbepoetin α

Das hier beispielhaft dargestellte erfindungsgemäße Reinigungsschema besteht aus drei chromatographischen Schritten mit üblichen Puffern.

In einem ersten Schritt wird ein EPO-Derivat Darbepoetin α enthaltender Überstand einer Fermentation mit biologischen Zellen mit Phosphatsalz auf eine Konzentration von 20 mM eingestellt, auf einen pH-Wert von 7,0 gepuffert und anschließend auf eine chromatographische Säule geladen, die mit CaptoBlue® gefüllt ist. Das EPO-Derivat Darbepoetin α bindet hierbei an das Säulenmaterial (d.h. an den Liganden Cibacron blue). Anschließend wird mit einem Waschpuffer (20 mM Phosphatpuffer, pH 7,0) gewaschen und das gebundene EPO-Derivat Darbepoetin α mit einem Elutionspuffer (20 mM Phosphatpuffer, 2 M NaCl, pH 7,0) eluiert.

Das Eluat wurde auf einen 20 mM Phosphatpuffer, pH 6,0, umgepuffert. Die Umpufferung erfolgte über eine Aufkonzentration (mit einer Amicon® Ultra-Filtrationsvorrichtung von Merck, MWCO = 10000) mit anschließender Verdünnung des Konzentrats mit 20 mM Phosphatpuffer, pH 6,0.

In einem zweiten Schritt wird dann das umgepufferte Eluat durch eine chromatographische Säule geführt, die mit einem Hydroxyapatitmaterial (z.B. Hydroxyapatitharz) gefüllt und mit 20 mM Phosphatpuffer, pH 6,0. equilibriert ist. Das EPO-Derivat Darbepoetin α bindet hierbei nicht an das Säulenmaterial (d.h. nicht an das Hydroxyapatitmaterial), sondern befindet sich in reinerer Form im Durchlauf. An das Säulenmaterial binden Verunreinigungen, die sich im Eluat aus dem ersten Schritt befinden und sich beispielsweise mit einem 500 mM Phosphatpuffer, pH 6.0, eluieren lassen. Das EPO-Derivat Darbepoetin α im Durchlauf liegt somit in reinerer Form vor als im Eluat aus dem ersten Schritt.

Der Durchlauf wurde auf einen 20 mM Trispuffer, pH 7,0, umgepuffert.

In dem dritten Schritt wird der umgepufferte Durchlauf auf eine chromatographische Säule geladen, die mit einem starken Anionenaustauschermaterial gefüllt und mit 20 mM Trispuffer, pH 7,0. equilibriert ist. Das EPO-Derivat Darbepoetin α bindet hierbei an das Säulenmaterial (d.h. an den starken Anionenaustauscher-Liganden). Anschließend wird mit einem Waschpuffer (20 mM Trispuffer, pH 7,0), bevorzugt mehreren Waschpuffern mit jeweils ansteigender Salzkonzentration (z.B. (20 mM Phosphatpuffer, pH 7,0, mit steigender NaCI-Konzentration) gewaschen, wodurch niedrig-sialyliertes Darbepoetin α weggewaschen wird (z.B. mit 20 mM Phosphatpuffer, 100 mM NaCl, pH 7,0) und hoch-sialyliertes Darbepoetin α an dem Säulenmaterial gebunden bleibt. Das gebundene hoch-sialylierte Darbepoetin α wird mit einem Elutionspuffer (20 mM Trispuffer, 500 mM NaCl, pH 7,0) eluiert. Durch das Wegwaschen der niedrig-sialylierten Spezies von Darbepoetin α kann somit eine Mischung von Darbepoetin α eluiert werden, die sich durch einen hohen Anteil an hoch-sialyierten Spezies von Darbepoetin α auszeichnet, wobei das Sialylierungsprofil des Darbepoetin α dem Sialylierungsprofil von Darbepoetin α entspricht, das im Stand der Technik bekannt ist und am Markt erhältlich ist (z.B. Darbepoetin α in Aranesp® des Herstellers Amgen) (siehe Figur).

## Patentansprüche

1. Verfahren zur Reinigung von EPO und/oder einem EPO-Derivat, umfassend
a) Kontaktieren einer Flüssigkeit enthaltend EPO und/oder ein EPO-Derivat mit einer festen Phase, die einen 1-Amino-2-Sulfo-Anthrachinon-Farbstoff gebunden hat, der 1-Amino-4-[4-[amino]-3-sulfoanilino]-9,10-dioxoanthracene-2-sulfonsäure enthält oder daraus besteht;
b) Waschen der festen Phase mit einer Waschflüssigkeit;
c) Elution des EPO und/oder EPO-Derivats von der festen Phase mit einer Elutionsflüssigkeit;
d) Kontaktieren einer Flüssigkeit, die aus der Elutionsflüssigkeit, die EPO und/oder das EPO-Derivat enthält, hervorgeht und einen Phosphatpuffer mit einem pH-Wert im Bereich von pH 5,5 bis 6,5 enthält, mit einem festen Hydroxyapatitmaterial und Sammeln des Durchlaufs, der das EPO und/oder das EPO-Derivat enthält;
e) Kontaktieren des Durchlaufs, der EPO und/oder das EPO-Derivat enthält, mit einem festen Anionenaustauschermaterial, wodurch das EPO(-Derivat) an das feste Anionenaustauschermaterial bindet;
f) Waschen des festen Anionenaustauschermaterials mit einer Waschflüssigkeit;
g) Elution des EPO und/oder EPO-Derivats von dem festen Anionenaustauschermaterial mit einer Elutionsflüssigkeit;
oder bestehend aus diesen Schritten, wobei in dem Verfahren keine Größenausschluss-Chromatographie durchgeführt wird.

2. Verfahren gemäß Anspruch 1, **dadurch gekennzeichnet, dass** der 1-Amino-2-Sulfo-Anthrachinon-Farbstoff
i) 1-Amino-4-[4-[[4-chloro-6-(2-sulfoanilino)-1,3,5-triazin-2-yl]amino]-3-sulfoanilino]-9,10-dioxoanthracene-2-sulfonsäure enthält oder daraus besteht; und/oder
ii) kovalent an die feste Phase gebunden ist; und/oder
iii) über ein Abstandshaltermolekül an die feste Phase gebunden ist, wobei das Abstandshaltermolekül bevorzugt über eine Etherbindung mit der festen Phase verbunden ist und/oder über eine Amidbindung mit der sulfonierten polyaromatischen Verbindung verbunden ist und insbesondere zwei bis vier Kohlenstoffatome und mindestens eine Hydroxygruppe aufweist.

3. Verfahren gemäß einem der vorhergehenden Ansprüche, **dadurch gekennzeichnet, dass** die feste Phase ein Material enthält oder daraus besteht, das ausgewählt ist aus der Gruppe bestehend aus Sephadex, Sepharose, Agarose, Polyacrylamid, Methacrylpolymer, Kieselerde und Kombinationen hiervon.

4. Verfahren gemäß einem der vorhergehenden Ansprüche, **dadurch gekennzeichnet, dass** das feste Anionenaustauschermaterial ein starkes Anionenaustauschermaterial ist.

5. Verfahren gemäß einem der vorhergehenden Ansprüche, **dadurch gekennzeichnet, dass** die Flüssigkeit enthaltend EPO und/oder das EPO-Derivat, die Waschflüssigkeit und/oder die Elutionsflüssigkeit
i) eine Puffersubstanz enthält, bevorzugt einen Phosphatpuffer; und/oder
ii) einen pH-Wert im Bereich von 5,0 bis 9,0, bevorzugt im Bereich von 5,5 bis 8,5, besonders bevorzugt im Bereich von 6,0 bis 8,0, insbesondere im Bereich von 6,5 bis 7,5, aufweist; und/oder
iii) maximal 10 mM L-Arginin, bevorzugt kein L-Arginin enthält.

6. Verfahren gemäß einem der vorhergehenden Ansprüche, **dadurch gekennzeichnet, dass** die
i) Flüssigkeit enthaltend EPO und/oder das EPO-Derivat ein nicht-pufferndes Salz enthält, bevorzugt NaCl und/oder KCl; und/oder
ii) Flüssigkeit enthaltend EPO und/oder das EPO-Derivat eine Fermentationsbrühe enthält oder daraus besteht, bevorzugt einen Überstand einer Fermentation mit biologischen Zellen, die das EPO und/oder EPO-Derivat produzieren, wobei die Fermentationsbrühe insbesondere über eine fluidische Verbindung zwischen dem Fermenter und der festen Phase vom Fermenter zur festen Phase geleitet wird; und/oder
iii) Waschflüssigkeit ein nicht-pufferndes Salz enthält, bevorzugt NaCl und/oder KCl; und/oder
iv) Elutionsflüssigkeit ein nicht-pufferndes Salz enthält, bevorzugt NaCl und/oder KCl.

7. Verfahren gemäß einem der vorhergehenden Ansprüche, **dadurch gekennzeichnet, dass** in dem Verfahren keine Umkehrphasen-Chromatographie durchgeführt wird.

8. Anlage zur Reinigung von EPO und/oder einem EPO-Derivat, enthaltend
a) eine feste Phase, wobei die feste Phase einen 1-Amino-2-Sulfo-Anthrachinon-Farbstoff gebunden hat, der 1-Amino-4-[4-[amino]-3-sulfoanilino]-9,10-dioxoanthracene-2-sulfonsäure enthält oder daraus besteht;
b) eine Flüssigkeit enthaltend EPO und/oder ein EPO-Derivat, die fluidisch, bevorzugt über ein Ventil, mit der festen Phase verbunden ist;
c) eine Waschflüssigkeit, die fluidisch, bevorzugt über ein Ventil, mit der festen Phase verbunden ist;
d) eine Elutionsflüssigkeit, die fluidisch, bevorzugt über ein Ventil, mit der festen Phase verbunden ist;
e) ein festes Hydroxyapatitmaterial;
f) ein festes Anionenaustauschermaterial;
g) eine Pumpe, die dazu geeignet ist, eine Flüssigkeit ausgewählt aus der Gruppe bestehend aus Flüssigkeit enthaltend das EPO-Derivat, Waschflüssigkeit, Elutionsflüssigkeit und Mischungen und Kombinationen hiervon, mit der festen Phase zu kontaktieren;
h) eine Steuereinheit, die dazu konfiguriert ist, zunächst die Flüssigkeit enthaltend das EPO-Derivat mit der festen Phase zu kontaktieren, anschließend die Waschflüssigkeit mit der festen Phase zu kontaktieren und dann die Elutionsflüssigkeit mit der festen Phase zu kontaktieren und das Eluat zu sammeln, wobei die Steuereinheit ferner dazu konfiguriert ist, eine Flüssigkeit, die fluidisch mit dem festen Hydroxyapatitmaterial verbunden ist, aus dem Eluat hervorgeht und einen Phosphatpuffer mit einem pH-Wert im Bereich von pH 5,5 bis 6,5 enthält, mit dem festen Hydroxyapatitmaterial zu kontaktieren und den Durchlauf, der EPO und/oder das EPO-Derivat enthält, zu sammeln, sowie den Durchlauf, der fluidisch mit dem festen Anionenaustauschermaterial verbunden ist, mit dem festen Anionenaustauschermaterial zu kontaktieren, das feste Anionenaustauschermaterial mit einer Waschflüssigkeit, die fluidisch mit dem festen Anionenaustauschermaterial verbunden ist, zu waschen, und mit einer Elutionsflüssigkeit, die fluidisch mit dem festen Anionenaustauschermaterial verbunden ist, das EPO und/oder EPO-Derivat von dem festen Anionenaustauschermaterial zu eluieren;
oder bestehend daraus, wobei die Anlage keine feste Phase zur Größenausschluss-Chromatographie enthält.

9. Anlage gemäß Anspruch 8, **dadurch gekennzeichnet, dass** der 1-Amino-2-Sulfo-Anthrachinon-Farbstoff
i) 1-Amino-4-[4-[[4-chloro-6-(2-sulfoanilino)-1,3,5-triazin-2-yl]amino]-3-sulfoanilino]-9,10-dioxoanthracene-2-sulfonsäure enthält oder daraus besteht; und/oder
ii) kovalent an die feste Phase gebunden ist; und/oder
iii) über ein Abstandshaltermolekül an die feste Phase gebunden ist, wobei das Abstandshaltermolekül bevorzugt über eine Etherbindung mit der festen Phase verbunden ist und/oder über eine Amidbindung mit der sulfonierten polyaromatischen Verbindung verbunden ist und insbesondere zwei bis vier Kohlenstoffatome und mindestens eine Hydroxygruppe aufweist.

10. Anlage gemäß einem der Ansprüche 8 oder 9, **dadurch gekennzeichnet, dass** das feste Anionenaustauschermaterial ein starkes Anionenaustauschermaterial ist.

11. Anlage gemäß einem der Ansprüche 8 bis 10, **dadurch gekennzeichnet, dass** die Flüssigkeit enthaltend EPO und/oder das EPO-Derivat, die Waschflüssigkeit und/oder die Elutionsflüssigkeit
i) eine Puffersubstanz enthält, bevorzugt einen Phosphatpuffer; und/oder
ii) einen pH-Wert im Bereich von 5,0 bis 9,0, bevorzugt im Bereich von 5,5 bis 8,5, besonders bevorzugt im Bereich von 6,0 bis 8,0, insbesondere im Bereich von 6,5 bis 7,5, aufweist; und/oder
iii) maximal 10 mM L-Arginin, bevorzugt kein L-Arginin enthält.

12. Anlage gemäß einem der Ansprüche 8 bis 11, **dadurch gekennzeichnet, dass** die
i) Flüssigkeit enthaltend EPO und/oder das EPO-Derivat ein nicht-pufferndes Salz enthält, bevorzugt NaCl und/oder KCl; und/oder
ii) Flüssigkeit enthaltend EPO und/oder das EPO-Derivat eine Fermentationsbrühe enthält oder daraus besteht, bevorzugt einen Überstand einer Fermentation mit biologischen Zellen, die das EPO und/oder EPO-Derivat produzieren, wobei Anlage insbesondere eine fluidische Verbindung zwischen dem Fermenter und der festen Phase aufweist, die dazu ausgebildet ist, die Fermentationsbrühe vom Fermenter zur festen Phase zu leiten; und/oder
iii) Waschflüssigkeit ein nicht-pufferndes Salz enthält, bevorzugt NaCl und/oder KCl; und/oder
iv) Elutionsflüssigkeit ein nicht-pufferndes Salz enthält, bevorzugt NaCl und/oder KCl.

13. Anlage gemäß einem der Ansprüche 8 bis 12, **dadurch gekennzeichnet, dass** die Anlage keine feste Phase zur Umkehrphasen-Chromatographie enthält.

## Claims

1. A method for purifying EPO and/or an EPO derivative, comprising
a) contacting a liquid containing EPO and/or an EPO derivative with a solid phase which has bound a 1-amino-2-sulfo-anthraquinone dye which contains or consists of 1-amino-4-[4-[amino]-3-sulfoanilino]-9,10-dioxoanthracene-2-sulfonic acid;
b) washing the solid phase with a washing liquid;
c) eluting the EPO and/or EPO derivative from the solid phase with an elution liquid;
d) contacting a liquid, which arises from the elution liquid which contains EPO and/or the EPO derivative and contains a phosphate buffer with a pH in the range from pH 5.5 to 6.5, with a solid hydroxyapatite material, and collecting the through feed which contains the EPO and/or the EPO derivative;
e) contacting the through feed which contains EPO and/or the EPO derivative with a solid anion exchanger material, as a result of which the EPO (derivative) binds to the solid anion exchanger material;
f) washing the solid anion exchanger material with a washing liquid;
g) eluting the EPO and/or EPO derivative from the solid anion exchanger material with an elution liquid;
or consisting of these steps, wherein no size-exclusion chromatography is carried out in the method.

2. A method according to Claim 1, **characterised in that** the 1-amino-2-sulfoanthraquinone dye
i) contains or consists of 1-amino-4-[4-[[4-chloro-6-(2-sulfoanilino)-1,3,5-triazin-2-yl]amino]-3-sulfoanilino]-9,10-dioxoanthracene-2-sulfonic acid; and/or
ii) is covalently bound to the solid phase; and/or
iii) is bound to the solid phase by way of a spacer molecule, the spacer molecule preferably being connected to the solid phase by way of an ether bond and/or being connected to the sulphonated polyaromatic compound by way of an amide bond and having in particular two to four carbon atoms and at least one hydroxyl group.

3. A method according to one of the preceding claims, **characterised in that** the solid phase contains or consists of a material which is selected from the group consisting of Sephadex, Sepharose, agarose, polyacrylamide, methacrylic polymer, silica and combinations thereof.

4. A method according to one of the preceding claims, **characterised in that** the solid anion exchanger material is a strong anion exchanger material.

5. A method according to one of the preceding claims, **characterised in that** the liquid containing EPO and/or the EPO derivative, the washing liquid and/or the elution liquid
i) contains a buffer substance, preferably a phosphate buffer; and/or
ii) has a pH in the range from 5.0 to 9.0, preferably in the range from 5.5 to 8.5, particularly preferably in the range from 6.0 to 8.0, in particular in the range from 6.5 to 7.5; and/or
iii) contains at most 10 mM L-arginine, preferably no L-arginine.

6. A method according to one of the preceding claims, **characterised in that** the
i) liquid containing EPO and/or the EPO derivative contains a non-buffering salt, preferably NaCl and/or KCl; and/or
ii) liquid containing EPO and/or the EPO derivative contains or consists of a fermentation broth, preferably a supernatant of a fermentation with biological cells which produce the EPO and/or EPO derivative, the fermentation broth being conducted from the fermenter to the solid phase in particular by way of a fluidic connection between the fermenter and the solid phase; and/or
iii) washing liquid contains a non-buffering salt, preferably NaCl and/or KCl; and/or
iv) elution liquid contains a non-buffering salt, preferably NaCl and/or KCl.

7. A method according to one of the preceding claims, **characterised in that** no reversed-phase chromatography is carried out in the method.

8. An installation for purifying EPO and/or an EPO derivative, containing
a) a solid phase, wherein the solid phase has bound a 1-amino-2-sulfoanthraquinone dye, which contains or consists of 1-amino-4-[4-[amino]-3-sulfoanilino]-9,10-dioxoanthracene-2-sulphonic acid;
b) a liquid containing EPO and/or an EPO derivative which is fluidically connected, preferably by way of a valve, to the solid phase;
c) a washing liquid which is fluidically connected, preferably by way of a valve, to the solid phase;
d) an elution liquid which is fluidically connected, preferably by way of a valve, to the solid phase;
e) a solid hydroxyapatite material;
f) a solid anion exchanger material;
g) a pump which is suitable for contacting a liquid selected from the group consisting of liquid containing the EPO derivative, washing liquid, elution liquid and mixtures and combinations thereof with the solid phase;
h) a control unit which is configured first of all to contact the liquid containing the EPO derivative with the solid phase, then to contact the washing liquid with the solid phase, and then to contact the elution liquid with the solid phase and to collect the eluate, wherein the control unit is further configured to contact a liquid, which is fluidically connected to the solid hydroxyapatite material, arises from the eluate and contains a phosphate buffer with a pH in the range from pH 5.5 to 6.5, with the solid hydroxyapatite material and to collect the through feed which contains EPO and/or the EPO derivative, and also to contact the through feed, which is fluidically connected to the solid anion exchanger material, with the solid anion exchanger material, to wash the solid anion exchanger material with a washing liquid which is fluidically connected with the solid anion exchanger material, and to elute the EPO and/or EPO derivative from the solid anion exchanger material with an elution liquid which is fluidically connected to the solid anion exchanger material;
or consisting thereof, wherein the installation does not contain any solid phase for size-exclusion chromatography.

9. An installation according to Claim 8, **characterised in that** the 1-amino-2-sulfoanthraquinone dye
i) contains or consists of 1-amino-4-[4-[[4-chloro-6-(2-sulfoanilino)-1,3,5-triazin-2-yl]amino]-3-sulfoanilino]-9,10-dioxoanthracene-2-sulfonic acid; and/or
ii) is covalently bound to the solid phase; and/or
iii) is bound to the solid phase by way of a spacer molecule, the spacer molecule preferably being connected to the solid phase by way of an ether bond and/or being connected to the sulphonated polyaromatic compound by way of an amide bond and having in particular two to four carbon atoms and at least one hydroxyl group.

10. An installation according to one of Claims 8 or 9, **characterised in that** the solid anion exchanger material is a strong anion exchanger material.

11. An installation according to one of Claims 8 to 10, **characterised in that** the liquid containing EPO and/or the EPO derivative, the washing liquid and/or the elution liquid
i) contains a buffer substance, preferably a phosphate buffer; and/or
ii) has a pH in the range from 5.0 to 9.0, preferably in the range from 5.5 to 8.5, particularly preferably in the range from 6.0 to 8.0, in particular in the range from 6.5 to 7.5; and/or
iii) contains at most 10 mM L-arginine, preferably no L-arginine.

12. An installation according to one of Claims 8 to 11, **characterised in that** the
i) liquid containing EPO and/or the EPO derivative contains a non-buffering salt, preferably NaCl and/or KCl; and/or
ii) liquid containing EPO and/or the EPO derivative contains or consists of a fermentation broth, preferably a supernatant of a fermentation with biological cells which produce the EPO and/or EPO derivative, wherein installation has in particular a fluidic connection between the fermenter and the solid phase which is designed to conduct the fermentation broth from the fermenter to the solid phase; and/or
iii) washing liquid contains a non-buffering salt, preferably NaCl and/or KCl; and/or
iv) elution liquid contains a non-buffering salt, preferably NaCl and/or KCl.

13. An installation according to one of Claims 8 to 12, **characterised in that** the installation does not contain any solid phase for reversed-phase chromatography.

## Revendications

1. Procédé de purification d'EPO et/ou d'un dérivé de l'EPO, comprenant
a) la mise en contact d'un liquide contenant de l'EPO et/ou un dérivé de l'EPO avec une phase solide, qui a lié un colorant de 1-amino-2-sulfo-anthraquinone, qui contient de l'acide 1-amino-4-[4-[amino]-3-sulfoanilino]-9,10-dioxoanthracène-2-sulfonique ou en est constitué ;
b) le lavage de la phase solide avec un liquide de lavage ;
c) l'élution de l'EPO et/ou du dérivé de l'EPO à partir de la phase solide à l'aide d'un liquide d'élution ;
d) la mise en contact d'un liquide qui dérive du liquide d'élution qui contient l'EPO et/ou le dérivé de l'EPO et contient un tampon phosphate ayant un pH compris dans la plage de pH 5,5 à 6,5, avec un matériau hydroxyapatite solide, et la collecte de l'éluat, qui contient l'EPO et/ou le dérivé de l'EPO ;
e) la mise en contact de l'éluat qui contient l'EPO et/ou le dérivé de l'EPO avec un matériau échangeur d'anions solide, ce qui provoque la liaison de l'EPO (ou du dérivé de l'EPO) au matériau échangeur d'anions solide ;
f) le lavage du matériau échangeur d'anions solide avec un liquide de lavage ;
g) l'élution de l'EPO et/ou du dérivé de l'EPO à partir du matériau échangeur d'anions solide avec un liquide d'élution ;
ou étant constitué de ces étapes, aucune chromatographie par exclusion de taille n'étant mise en œuvre dans le procédé.

2. Procédé selon la revendication 1, **caractérisé en ce que** le colorant de 1-amino-2-sulfo-anthraquinone
i) contient, ou en est constitué, de l'acide 1-amino-4-[4[[4-chloro-6-(2-sulfoanilino)-1,3,5-triazin-2-yl]amino]-3-sulfoanilino]-9,10-dioxoanthracène-2-sulfonique ; et/ou
ii) est lié par liaison covalente à la phase solide ; et/ou
iii) est lié à la phase solide par l'intermédiaire d'une molécule intercalaire, la molécule intercalaire étant de préférence reliée à la phase solide par l'intermédiaire d'une liaison éther et/ou étant reliée par l'intermédiaire d'une liaison amide au composé polyaromatique sulfoné, et en particulier contenant deux à quatre atomes de carbone et au moins un groupe hydroxy.

3. Procédé selon l'une des revendications précédentes, **caractérisé en ce que** la phase solide contient, ou en est constituée, un matériau qui est choisi dans le groupe consistant en le Sephadex, la Sepharose, l'Agarose, le polyacrylamide, un polymère méthacrylique, la silice et les combinaisons de ceux-ci.

4. Procédé selon l'une des revendications précédentes, **caractérisé en ce que** le matériau échangeur d'anions solide est un matériau échangeur d'anions fort.

5. Procédé selon l'une des revendications précédentes, **caractérisé en ce que** le liquide contenant l'EPO et/ou le dérivé de l'EPO, le liquide de lavage et/ou le liquide d'élution
i) contiennent une substance tampon, de préférence un tampon phosphate ; et/ou
ii) présentent un pH compris dans la plage de 5,0 à 9,0, de préférence dans la plage de 5,5 à 8,5, d'une manière particulièrement préférée dans la plage de 6,0 à 8,0, en particulier dans la plage de 6,5 à 7,5 ; et/ou
iii) contiennent au maximum 10 mM de L-arginine et de préférence ne contiennent pas de L-arginine.

6. Procédé selon l'une des revendications précédentes, **caractérisé en ce que**
i) le liquide contenant l'EPO et/ou le dérivé de l'EPO contient un sel non tamponnant, de préférence NaCl et/ou KCI ; et/ou
ii) le liquide contenant l'EPO et/ou le dérivé de l'EPO contient un bouillon de fermentation ou en est constitué, de préférence un surnageant d'une fermentation avec des cellules biologiques qui produisent l'EPO et/ou le dérivé de l'EPO, le bouillon de fermentation étant en particulier envoyé du fermenteur à la phase solide par l'intermédiaire d'une liaison fluidique entre le fermenteur et la phase solide ; et/ou
iii) le liquide de lavage contient un sel non tamponnant, de préférence NaCl et/ou KCI ; et/ou
iv) le liquide d'élution contient un sel non tamponnant, de préférence NaCl et/ou KCI.

7. Procédé selon l'une des revendications précédentes, **caractérisé en ce qu'**aucune chromatographie en phase inverse n'est mise en œuvre dans le procédé.

8. Installation pour la purification d'EPO et/ou d'un dérivé d'EPO, contenant
a) une phase solide qui a lié un colorant de 1-amino-2-sulfo-anthraquinone, qui contient, ou en est constitué, de l'acide 1-amino-4-[4-[amino]-3-sulfoanilino]-9,10-dioxoanthracène-2-sulfonique ;
b) un liquide contenant de l'EPO et/ou un dérivé de l'EPO, qui est relié à la phase solide par une liaison fluidique, de préférence par une vanne ;
c) un liquide de lavage, qui est relié à la phase solide par une liaison fluidique, de préférence par une vanne ;
d) un liquide d'élution, qui est relié à la phase solide par une liaison fluidique, de préférence par une vanne ;
e) un matériau hydroxyapatite solide ;
f) un matériau échangeur d'anions solide ;
g) une pompe, qui permet de mettre en contact avec la phase solide un liquide choisi dans le groupe consistant en le liquide contenant le dérivé de l'EPO, le liquide de lavage, le liquide d'élution, et les mélanges et combinaisons de ceux-ci ;
h) une unité de commande, qui est configurée pour d'abord mettre en contact le liquide contenant le dérivé de l'EPO avec la phase solide, puis pour mettre en contact le liquide de lavage avec la phase solide, puis pour mettre en contact le liquide d'élution avec la phase solide et collecter l'éluat, l'unité de commande étant en outre configurée pour mettre en contact avec le matériau hydroxyapatite solide un liquide qui est relié par une liaison fluidique au matériau hydroxyapatite solide, provient de l'éluat et contient un tampon phosphate ayant un pH compris dans la plage de pH 5,5 à 6,5, et pour collecter l'éluat qui contient l'EPO et/ou le dérivé de l'EPO, et aussi pour mettre en contact avec le matériau échangeur d'anions solide l'éluat qui est relié par une liaison fluidique au matériau échangeur d'anions solide, pour laver le matériau échangeur d'anions solide avec un liquide de lavage qui est relié par liaison fluidique au matériau échangeur d'anions solide, et pour éluer l'EPO et/ou le dérivé de l'EPO à partir du matériau échangeur d'anions solide à l'aide d'un liquide d'élution qui est relié par une liaison fluidique au matériau échangeur d'anions solide ;
ou en étant constituée, l'installation ne contenant aucune phase solide pour chromatographie par exclusion de taille.

9. Installation selon la revendication 8, **caractérisée en ce que** le colorant de 1-amino-2-sulfo-anthraquinone
i) contient, ou en est constitué, de l'acide 1-amino-4-[4[[4-chloro-6-(2-sulfoanilino)-1,3,5-triazin-2-yl]amino]-3-sulfoanilino]-9,10-dioxoanthracène-2-sulfonique ; et/ou
ii) est lié par liaison covalente à la phase solide ; et/ou
iii) est lié à la phase solide par l'intermédiaire d'une molécule intercalaire, la molécule intercalaire étant de préférence reliée à la phase solide par une liaison éther, et/ou étant reliée au composé polyaromatique sulfoné par une liaison amide et en particulier présentant deux à quatre atomes de carbone et au moins un groupe hydroxy.

10. Installation selon l'une des revendications 8 ou 9, **caractérisée en ce que** le matériau échangeur d'anions solide est un matériau échangeur d'anions fort.

11. Installation selon l'une des revendications 8 à 10, **caractérisée en ce que** le liquide contenant l'EPO et/ou le dérivé de l'EPO, le liquide de lavage et/ou le liquide d'élution
i) contiennent une substance tampon, de préférence un tampon phosphate ; et/ou
ii) présentent un pH compris dans la plage de 5,0 à 9,0, de préférence dans la plage de 5,5 à 8,5, d'une manière particulièrement préférée dans la plage de 6,0 à 8,0, en particulier dans la plage de 6,5 à 7,5 ; et/ou
iii) contiennent au maximum 10 mM de L-arginine et de préférence ne contiennent pas de L-arginine.

12. Installation selon l'une des revendications 8 à 11, **caractérisée en ce que**
i) le liquide contenant l'EPO et/ou le dérivé de l'EPO contient un sel non tamponnant, de préférence NaCl et/ou KCl ; et/ou
ii) le liquide contenant l'EPO et/ou le dérivé de l'EPO contient un bouillon de fermentation ou en est constitué, de préférence un surnageant d'une fermentation avec des cellules biologiques qui produisent l'EPO et/ou le dérivé de l'EPO, l'installation présentant en particulier une liaison fluidique entre le fermenteur et la phase solide, qui est conçue pour envoyer le bouillon de fermentation du fermenteur à la phase solide ; et/ou
iii) le liquide de lavage contient un sel non tamponnant, de préférence NaCl et/ou KCl ; et/ou
iv) le liquide d'élution contient un sel non tamponnant, de préférence NaCl et/ou KCl.

13. Installation selon l'une des revendications 8 à 12, **caractérisée en ce que** l'installation ne contient aucune phase solide pour chromatographie en phase inversée.
